# EUROPEAN PATENT APPLICATION

(11) **EP 2 301 560 A1**
(43) Date of publication of application: **30.03.2011**
(21) Application number: 09738877.1
(22) Date of filing: 30.04.2009
(51) Int. Cl.: A61K 38/00, A61K 31/7088, A61K 35/76, A61K 48/00, A61P 17/02, A61P 43/00, C07K 14/47, C12N 5/00, C12N 15/09

(54) **PHARMACEUTICAL AGENT FOR PROMOTING THE FUNCTIONAL REGENERATION OF DAMAGED TISSUE**

(30) Priority: 30.04.2008 JP 2008119324
(71) Applicant: Genomix Co., Ltd., Ibaraki-shi Osaka 567-0085 (JP); Osaka University, Suita-shi Osaka 565-0871 (JP)
(72) Inventor: TAMAI, Katsuto, Suita-shi Osaka 565-0871 (JP); YAMAZAKI, Takehiko, Minoo-shi Osaka 562-0031 (JP); CHINO, Takenao, Minoo-shi Osaka 562-0031 (JP); KANEDA, Yasufumi, Suita-shi Osaka 565-0871 (JP)
(74) Representative: Plougmann & Vingtoft A/S
(86) International application number: PCT/JP2009/058519
(87) International publication number: WO 2009/133940

(57) **Abstract**

The present inventors assessed the possibility that bone marrow-derived cells are mobilized to the grafted skin from nonskin tissues and contribute to skin tissue regeneration during the engraftment of grafted skin on biological tissues. As a result, the present inventors for the first time in the world demonstrated that:
(1) a large number of bone marrow-derived cells are mobilized to grafted skin;
(2) mobilized bone marrow-derived cells differentiate into any of dermal fibroblasts, adipocytes, muscle cells, vascular endothelial cells, and epidermal keratinocytes in grafted skin, and thus mobilized bone marrow-derived cells include bone marrow-derived mesenchymal stem cells;
(3) S 100A8 and S 100A9 released from necrotic tissues of grafted skin are responsible for mobilizing bone marrow-derived mesenchymal stem cells to the grafted skin from peripheral blood; and
(4) purified S100A8 and S100A9 promote the migration of mesenchymal stem cells isolated/cultured from the bone marrow.

## Description

### Technical Field

The present invention relates to pharmaceuticals that promote functional regeneration of damaged tissues.

### Background Art

In recent years, it has been revealed that various stem cells contribute towards the repairing process of damaged tissues, and development of novel regenerative medicines that induce functional tissue regeneration by mobilizing a large number of stem cells to lesion sites is in progress. To bring these novel regenerative medicines to realization, (i) stem cells that are mobilizable to lesion sites must be present *abundantly in vivo;* and (ii) factors that mobilize stem cells to lesion sites must be isolated/identified.

Examples of stem cells that are mobilizable to lesion sites include tissue stem cells present in lesion areas or nearby tissues, and bone marrow-derived stem cells present in peripheral blood. In recent years, it has been reported that bone marrow-derived cells contribute to many types of damaged tissue regenerations, but the mechanism for mobilizing bone marrow-derived cells to lesion sites is unknown. Bone marrow-derived cells as used herein are distinguished from hematopoietic stem cells which have the potential to differentiate into blood cells (leukocytes and erythrocytes), and include stem cells represented by cells called bone marrow mesenchymal stem cells, or tissue progenitor cell groups present in the bone marrow. Bone marrow mesenchymal stem cells are undifferentiated stem cells with the potential to differentiate into osteoblasts, adipocytes, and chondrocytes, and can further differentiate into other mesenchymal cells such as fibroblasts, muscle cells, stromal cells, and tendon cells. Recently, it has been proved that bone marrow mesenchymal stem cells differentiate into nerve cells and further to epithelial cells (such as skin keratinocytes) and vascular endothelial cells (Non-patent Document 1). Tissue progenitor cells are defined as undifferentiated cells having a unidirectional potential to differentiate into specific tissues/cells other than those of the blood system, and include undifferentiated cells with the potential to differentiate into mesenchymal tissue, epithelial tissue, nerve tissue, parenchymatous organs, and vascular endothelium, as mentioned above.

The S100 protein family consists of about 20 types of proteins, most of which are present in the cytoplasm, but some of which are known to be secreted outside the cell and play various functions at the time of tissue damage, skin cancer, and inflammation. The proteins also play important roles in the epidermal cell differentiation in the skin. In particular, the expression of S100A8 and S 100A9 is known to be strongly induced in about one week after skin damage (Non-patent Document 2). The S100A8/A9 (or MRP-8/14) heterodimer has an affinity for heparin (Non-patent Document 3). S100A8, S100A9, and the S100A8/A9 heterodimer have the activity of inducing the migration of neutrophils to inflammatory sites (Non-patent Document 4).

Conventionally it was thought that central nerve cells in the brain and spinal cord cannot be regenerated once damaged. However, recently the existence of neural stem cells became known and induction of these cells was made possible. The neural stem cell niche within the normal nerve system has also been identified. Therefore, recovery of damaged central neurons, which was long considered impossible, is now expected to be feasible. Currently, research related to neuronal regeneration for brain and spinal cord injury, degenerative diseases, and the like is being expanded.

The main causes of brain tissue (cells) injury are traumatic cerebral contusion and cerebral ischemic diseases. Other causes can be injury resulting from brain surgeries such as brain tumor removal. In particular, complete removal of neuroglioma that have developed from cerebral parenchymal cells is difficult, and there is no choice but to stop at partial removal to avoid damage to motor and language functions. Moreover, malignant neuroglioma has a worse prognosis, and none of the treatments of active research in recent years ranging from chemotherapy and radiotherapy to immunotherapy/gene therapy has achieved satisfactory effects. Accordingly, an ideal treatment would be one that can remove as many tumor cells as possible, and restore damage to cerebral functions that results from the removal.

### [Prior Art Documents]

### [Non-patent Documents]

[Non-patent Document 1] Wu *Y et al., Stem Cells. 2007 25(10): 2648-2659
[Non-patent Document 2] Eckert RL et al., J. Invest Dermatol. 2004 Jul; 123(1): 23-33
[Non-patent Document 3] Robinson MJ et al., J Biol Chem. 2002 Feb 1; 277(5): 3658-65. Epub
[Non-patent Document 4] Ryckman et al., J. Immunol. 2003 Mar 15; 170(6): 3233-42

### Disclosure of the Invention

### [Problems to be Solved by the Invention]

An objective of the present invention is to isolate/identify factors mobilizing cells that differentiate into damaged tissues to lesion sites, and to provide inventions utilizing such factors.

### [Means for Solving the Problems]

Once factors that mobilize cells differentiating into damaged tissues are revealed, the administration of such factors to the body allows the mobilization of a large number of cells (which exist in peripheral blood or local tissues) that differentiate into damaged tissues. Thus, the development of novel regenerative medicines which promote functional tissue regeneration is made possible.

The present inventors examined the possibility that bone marrow-derived cells might be mobilized to skin grafts from non-skin tissues in the process of skin graft transplantation into living tissue, thus contributing to skin tissue regeneration. As a result, they revealed the following for the first time in the world.
1) a large amount of bone marrow-derived cells are mobilized to the grafted skin;
2) the mobilized bone marrow-derived cells are differentiated into any of dermal fibroblasts, adipocytes, muscle cells, vascular endothelial cells, and epidermal keratinocytes in the grafted skin, and mobilized bone marrow-derived cells include bone marrow-derived mesenchymal stem cells;
3) the factors which mobilize bone marrow-derived mesenchymal stem cells from peripheral blood to the grafted skin are S 100A8 and S 100A9 released from the necrosed tissue of recipient skin; and
4) purified S 100A8 and S 100A9 promote the migration of mesenchymal stem cells isolated and cultured from bone marrow. Based on these findings, the present application provides the following inventions:
   [1] a bone marrow cell-inducing agents, which comprise a component of any of (a) to (f) described below:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein;
   [2] a tissue regeneration-promoting agents, which comprise the component of any of (a) to (f) described below:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein;
   [3] a method for inducing bone marrow cells to a damaged tissue, which comprise the step of administering any one of the following materials of (a) to (f) to a subject whose tissue is damaged:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein;
   [4] a method for promoting a damaged tissue regeneration, which comprise the step of administering any one of the following materials of (a) to (f) to a subject whose tissue is damaged:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein;
   [5] use of a material of any one of the following (a) to (f) in the manufacture of a bone marrow cell-inducing agent:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein;
   [6] use of a material of any one of the following (a) to (f) in the manufacture of a tissue regeneration-promoting agent:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein;
   [7] a material of any one of the following (a) to (f) for use in a method for inducing a bone marrow cell to a damaged tissue:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein; and
   [8] a material of any one of the following (a) to (f) for use in a method for promoting tissue regeneration of a damaged tissue:
      (a) an S100A8 protein;
      (b) a cell that secretes an S 100A8 protein;
      (c) a vector inserted with a DNA encoding an S 100A8 protein;
      (d) an S 100A9 protein;
      (e) a cell that secretes an S 100A9 protein; and
      (f) a cell that secretes an S 100A9 protein.

### Brief Description of the Drawings

Fig. 1 shows in a diagram a method for producing GFP bone marrow-transplanted mice.
Fig. 2 shows in a set of photographs the accumulation of GFP fluorescence in grafted skin observed after skin is grafted onto the back of GFP bone marrow-transplanted mice. The left photograph (A) shows nuclear staining with DAPI. The middle photograph (B) shows green fluorescence of GFP-positive bone marrow-derived cells accumulated at the skin graft site. The right photograph (C) shows a merged image of photographs (A) and (B). Bone marrow-derived cells are reconstructing skin tissues.

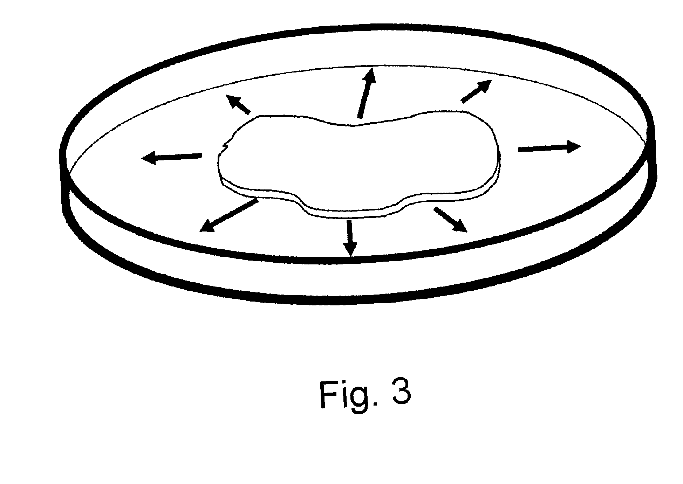

Fig. 3 shows in a diagram a method for extracting regeneration-inducing factors from an excised skin piece.
Fig. 4 shows in a photograph assay results of measuring the migratory activity of bone-marrow derived mesenchymal stem cells in skin extracts using a Boyden chamber. These images show blue-stained bone marrow mesenchymal stem cells, which have migrated from the upper compartment of the Boyden chamber through a 8-µm micropore polycarbonate membrane filter into the lower compartment containing skin extracts, and adhered to the lower-compartment side of the membrane. Skin extracts collected from two-day-old or six-week-old mice were placed in the lower chambers.
Fig. 5 shows in a set of photographs Western blot detection of the S100A8 and S100A9 proteins in skin extracts.
Fig. 6 shows in a photograph elution of a heparin-binding protein in skin extracts eluted from a heparin affinity column by a concentration gradient of NaCl. Proteins in each fraction were separated by SDS-PAGE and detected by silver staining.
Fig. 7 shows in a photograph assay results of measuring the migratory activity of bone marrow-derived mesenchymal stem cells in skin extracts using a Boyden chamber. The image shows blue-stained bone marrow mesenchymal stem cells, which have migrated from the upper compartment of the Boyden chamber through the micropores of a filter to each heparin-binding fraction in skin extracts (to the lower compartment), and adhered to the lower-compartment side of the membrane.
Fig. 8 shows in a set of photographs Western blot detection of the S100A8 and S100A9 proteins in each heparin-binding fraction of skin extracts.
Fig. 9 shows in a diagram the expression vector for S100A8 or S100A9.
Fig. 10 shows in a photograph assay results of measuring the migratory activity of bone marrow-derived mesenchymal stem cells in skin extracts using a Boyden chamber. These images show blue-stained bone marrow mesenchymal stem cells, which have migrated from the upper compartment of the Boyden chamber through the micropores of a filter into the lower compartment containing recombinant GST-S100A8, GST-S100A9, or skin extracts, and adhered to the lower-compartment side of the membrane.
Fig. 11A presents a set of diagrams showing a FACS result for CD44, PDGFRα, and PDGFRβ in the CD45-negative cell fraction in peripheral blood 12 hours after administration of GST-S 100A8 or GST-S 100A9 *via* the mouse caudal vein. Fig. 11B presents a set of graphs by quantitatively analyzing the population of CD45-negative, CD44-positive, PDGFRα-positive cells (left), or CD45-negative, CD44-positive, PDGFRβ-positive cells (right).
Fig. 12 shows in a graph therapeutic effect of S100A8 on cutaneous ulcer in normal mice.
Fig. 13 shows in a graph therapeutic effect of S 100A8 on cutaneous ulcer in diabetic mice.

### Mode for Carrying Out the Invention

The present invention provides bone marrow cell-inducing agents, which comprise at least one of the components of (a) to (f) described below:
(a) an S100A8 protein;
(b) a cell that secretes an S 100A8 protein;
(c) a vector inserted with a DNA encoding an S 100A8 protein;
(d) an S 100A9 protein;
(e) a cell that secretes an S 100A9 protein; and
(f) a cell that secretes an S 100A9 protein.

Using the inducing agents, functional tissue regeneration can be promoted by inducing bone marrow-derived cells locally (near the sites of administration or application of the above ingredients, or damaged sites). Thus, the inducing agents can be used as an essential reagent in basic and clinical studies for developing regeneration medicine and regeneration-inducing medicine. For example, it is possible to assess in an experimental animal the mobilization of bone marrow-derived cells to essential biological tissues and the degree of tissue repair or functional tissue reconstruction. In addition, induction of tissue regeneration can be studied by *in vitro* mobilization of bone marrow-derived cells.

Furthermore, regeneration of damaged tissues can be promoted by using the inducing agents. Furthermore, the above-described inducing agents are expected to be used not only as an inducer/promoter of functional tissue regeneration but also as a so-called preventive drug to prevent the functional impairment of tissues/organs caused by reduction in the number of tissue stem cells or as an anti-aging drug to delay the progression of age-related changes.

The inducing agents of the present invention include agents for mobilizing bone marrow cells to peripheral blood from bone marrow, which comprise any one of the components of (a) to (f) described below:
(a) an S100A8 protein;
(b) a cell that secretes an S 100A8 protein;
(c) a vector inserted with a DNA encoding an S 100A8 protein;
(d) an S 100A9 protein;
(e) a cell that secretes an S 100A9 protein; and
(f) a cell that secretes an S 100A9 protein.

The above-described agents are administered to blood vessel or muscle.

Such agents for mobilizing bone marrow cells to peripheral blood from bone marrow mobilize bone marrow cells to peripheral blood from bone marrow when administered to blood vessel or muscle. Once mobilized to peripheral blood, bone marrow cells are induced to damaged tissues from peripheral blood.

Thus, functional tissue regeneration can be promoted by using the agents to induce bone marrow cells to damaged sites. Thus, the agents can be used as an essential reagent in basic and clinical studies for developing regeneration medicine and regeneration-inducing medicine.

Furthermore, regeneration of damaged tissues can be promoted by using the above-described agents. In particular, the above-described agents induce bone marrow-derived cells to damaged sites when administered to blood vessel or muscle. Thus, the above-described agents enable promotion of functional tissue regeneration even in tissue damages of brain and heart where it is difficult to directly administer agents from outside of the body.

The present invention also provides agents and kits for promoting tissue regeneration, which comprise at least one of the components of (a) to (f) described below:
(a) an S100A8 protein;
(b) a cell that secretes an S 100A8 protein;
(c) a vector inserted with a DNA encoding an S 100A8 protein;
(d) an S 100A9 protein;
(e) a cell that secretes an S 100A9 protein; and
(f) a cell that secretes an S 100A9 protein.

The agents or kits for promoting tissue regeneration induce blood-circulating bone marrow-derived cells to the damaged tissues (local induction) from peripheral blood, when administered to damaged tissues or near the damaged sites, or to blood vessel or muscle.

Moreover, examples of kits for promoting tissue regeneration include: kits for promoting tissue regeneration comprising (1) the above-mentioned components dissolved in fibrinogen, and (2) thrombin; or alternatively, kits for promoting tissue regeneration comprising (1) the above-mentioned components, (2) fibrinogen, and (3) thrombin. In the present invention, commercially available fibrinogen and thrombin can be used. Examples include, but are not limited to, fibrinogen HT-Wf (Benesis-Mitsubishi Pharma), Beriplast (ZLB Behring), Tisseel (Baxter), Bolheal (Kaketsuken), and TachoComb (ZLB Behring).

The type of tissue to be regenerated is not particularly limited. The tissue may be any tissue, as long as it is a damaged tissue. Such tissues include, for example, live skin tissues and tissues obtained by biopsy (surgery) from the body (brain, lung, heart, liver, stomach, small and large intestines, pancreas, kidney, urinary bladder, spleen, uterus, testis, blood, etc.). In particular, the agents of the present invention are efficiently used to regenerate tissues (brain, heart, etc.) that are difficult for direct administration of agents from outside of the body.

Bone marrow-derived cells that are mobilized to the damaged tissue differentiate into various types of cells to contribute to functional regeneration of the damaged tissue and maintenance/enhancement of the functions. In the present invention, examples of damaged tissue include, but are not limited to, tissues damaged by various pathological conditions, trauma, burns, inflammation, autoimmunity, gene abnormalities, and the like causing ischemic/hypoperfusive/hypoxic conditions. Damaged tissue also includes necrosed tissues.

Tissues in the present invention are not particularly limited as long as bone marrow-derived cells can differentiate into the tissues. All types of tissues in the living body can be exemplified, such as skin tissue, bone tissue, cartilage tissue, muscle tissue, adipose tissue, cardiac muscle tissue, neurological tissue, pulmonary tissue, gastrointestinal tissues, hepatic/biliary/pancreatic tissues, and genitourinary organs. Moreover, with use of the above tissue regeneration-promoting agents, treatments for inducing functional tissue regeneration becomes possible not only in cutaneous diseases such as intractable cutaneous ulcers, skin wounds, bullosis, and alopecia, but also in tissue damages such as cerebral infarction, myocardial infarction, bone fracture, pulmonary infarction, gastric ulcers, and enteritis. The types of animals to be administered with the above tissue regeneration-promoting agents include human and non-human animals, which can be exemplified by, but are not limited to, humans, mice, rats, monkeys, pigs, dogs, rabbits, hamsters, and guinea pigs.

Furthermore, the agents of the present invention can be administered to diabetes patients. It is known that intractable skin ulcer which is a skin complication of diabetes is difficult to treat as compared to skin ulcer in normal persons. The agents of the present invention are also efficiently used for such diabetes patients.

Bone marrow cells of the present invention are cells other than hematopoietic stem cells, or cells derived therefrom such as leukocytes, erythrocytes, and platelets, and include stem cells represented by cells which have been hitherto called bone marrow mesenchymal stem cells, bone marrow stromal pluripotent stem cells, or bone marrow pluripotent stem cells and tissue progenitor cell populations existing in the bone marrow. Bone marrow cells of the present invention can be isolated from bone-marrow extracts (bone marrow cell extracts) or peripheral blood collection. Hematopoietic stem cells are nonadherent, while bone marrow cells of the present invention are obtained as adherent cells by means of a cell culture of a mononuclear cell fraction of blood obtained from the bone marrow extracts (bone marrow cell extracts) or peripheral blood collection. Moreover, bone marrow cells of the present invention include mesenchymal stem cells, and have a potential to differentiate into, preferably, osteoblasts (the induction of differentiation can be identified by observing calcification), chondrocytes (which can be identified by alcian blue positive staining, safranin O positive staining, or the like), adipocytes (which can be identified by Sudan III positive staining), and other mesenchymal cells such as fibroblasts, smooth muscle cells, stromal cells, and tendon cells; and further nerve cells, epithelial cells (for example, epidermal keratinocytes and intestinal epithelial cells express cytokeratin family), and vascular endothelial cells. However, the cells to be differentiated into are not limited to the above cells, and the potential to differentiate into cells of parenchymatous organs such as liver, kidney, and pancreas are also included.

In the present invention, bone marrow-derived mesenchymal stem cells, bone marrow stromal pluripotent stem cells, or bone marrow pluripotent stem cells refer to cells existing in the bone marrow, which are directly collected from the bone marrow or indirectly collected from other tissues (blood, skin, adipose, and other tissues), and can be cultured/proliferated as adherent cells on a culture dish (made of plastic or glass). These cells are characterized in having a potential to differentiate into mesenchymal tissues (mesenchymal stem cells) such as bone, cartilage, and adipose, or skeletal muscles, heart muscles, further, nerve tissues, epithelial tissues (pluripotent stem cells) and can be obtained from a collection of bone marrow blood, peripheral blood, or mesenchymal tissues such as adipose, epithelial tissues such as skin, nerve tissues such as brain. Bone marrow-derived mesenchymal stem cells, bone marrow-derived pluripotent stem cells, or bone marrow pluripotent stem cells are also characterized in having a potential to differentiate into epithelial tissues such as keratinocytes that constitute skin or into nerve tissues that constitute brain, by administrating these cells that have once adhered onto a culture dish to a lesion area of the living body.

Bone marrow mesenchymal stem cells, bone marrow stromal pluripotent stem cells, or bone marrow pluripotent stem cells of the present invention are multipotent stem cells, and have a potency to differentiate preferably into: osteoblasts (the induction of differentiation can be identified by observing calcification), chondrocytes (which can be identified by alcian blue positive staining, safranin O positive staining, or the like), adipocytes (which can be identified by Sudan III positive staining or the like), and other mesenchymal cells such as fibroblasts, smooth muscle cells, skeletal muscle cells, stromal cells, and tendon cells; nerve cells, pigment cells, epidermal cells, hair follicle cells (which express cytokeratin family, hair keratin family, or the like), epithelial cells (for example, epidermal keratinocytes and intestinal epithelial cells express cytokeratin family or the like), and endothelial cells; and further preferably into cells of parenchymatous organs such as liver, kidney, and pancreas. However, differentiated cells are not limited to the above cells.

Moreover, human bone marrow mesenchymal stem cells, bone marrow stromal pluripotent stem cells, or bone marrow pluripotent stem cells can be exemplified by, but are not limited to, cells which can be directly obtained from collecting bone marrow (bone marrow cell extracts), peripheral blood, or adipose, or obtained as adherent cells through culturing of an isolated mononuclear cell fraction. Markers for human bone marrow mesenchymal stem cells, bone marrow stromal pluripotent stem cells, or bone marrow pluripotent stem cells can be, for example, all or some of the markers of Lin-negative, CD45-negative, and CD44-positive, but are not limited to.

Moreover, mouse bone marrow mesenchymal stem cells, bone marrow stromal pluripotent stem cells, or bone marrow pluripotent stem cells can be exemplified by, but are not limited to, cells which can be obtained by methods described in the Examples. Markers for mouse bone marrow mesenchymal stem cells, bone marrow stromal pluripotent stem cells, or bone marrow pluripotent stem cells can be for example, all or some of the markers of CD44-positive, PDGFRα-positive, PDGFRβ-positive, CD45-negative, Lin-negative, Sca-1 positive, and c-kit negative, but are not limited to.

Tissue progenitor cells are defined as undifferentiated cells having a unidirectional potency to differentiate into specific tissue cells other than the blood system, and include undifferentiated cells having the potency to differentiate into mesenchymal tissue, epithelial tissue, nerve tissue, parenchymatous organs, and vascular endothelium as mentioned above.

In the inducing agents and tissue regeneration-promoting agents of the present invention, the components besides at least one of the components (a) to (f) described above are not particularly limited, as long as the components do not inhibit the induction of bone marrow-derived cells and promotion of tissue regeneration. For example, tissue regeneration-promoting agents of the present invention may comprise, in addition to at least one of the components (a) to (f) described above, related molecule(s) that promote the activity of S 100A8 or S 100A9 to induce functional tissue regeneration, related molecule(s) that suppress activities other than the expected activity of S 100A8 or S 1 00A9, factors that regulate the proliferation and differentiation of bone marrow-derived cells, and factors that maintain/enhance the function of the above factors or cells.

The types of animals which serve as a source for S100A8 or S100A9 proteins in the inducing agents and tissue regeneration-promoting agents of the present invention include human and non-human animals, which can be exemplified by humans, mice, rats, monkeys, pigs, dogs, rabbits, hamsters, and guinea pigs, but the type of animal is preferably the same as the animal to be administered with the above ingredients.

The S 100A8 protein in inducing agents or tissue regeneration-promoting agents of the present invention can be exemplified by, but is not limited to, proteins comprising the amino acid sequence of SEQ ID NO: 1, 3, or 5. The S100A8 protein of the present invention can also include proteins which are functionally equivalent to the protein comprising the amino acid sequence of SEQ ID NO: 1, 3, or 5. Examples of such proteins include: 1) isolated proteins which comprise an amino acid sequence with one or more amino acid substitutions, deletions, insertions, and/or additions in the amino acid sequence of SEQ ID NO: 1, 3, or 5, and which are functionally equivalent to the protein comprising the amino acid sequence of SEQ ID NO: 1, 3, or 5; and 2) isolated proteins which are encoded by DNAs that hybridize under stringent conditions with DNAs comprising the nucleotide sequence of SEQ ID NO: 2, 4, or 6, and which are functionally equivalent to the protein comprising the amino acid sequence of SEQ ID NO: 1, 3, or 5.

The S 100A9 protein in inducing agents or tissue regeneration-promoting agents of the present invention can be exemplified by, but is not limited to, proteins comprising the amino acid sequence of SEQ ID NO: 7, 9, or 11. The S100A9 protein of the present invention can also include proteins which are functionally equivalent to the protein comprising the amino acid sequence of SEQ ID NO: 7, 9, or 11. Examples of such proteins include: 1) isolated proteins which comprise an amino acid sequence with one or more amino acid substitutions, deletions, insertions, and/or additions in the amino acid sequence of SEQ ID NO: 7, 9, or 11, and which are functionally equivalent to the protein comprising the amino acid sequence of SEQ ID NO: 7, 9, or 11; and 2) isolated proteins which are encoded by DNAs that hybridize under stringent conditions with DNAs comprising the nucleotide sequence of SEQ ID NO: 8, 10, or 12, and which are functionally equivalent to the protein comprising the amino acid sequence of SEQ ID NO: 7, 9, or 11.

Isolated proteins which are functionally equivalent to the protein comprising the amino acid sequence of SEQ ID NO: 1, 3, 5, 7, 9, or 11 may be homologues or paralogues to the protein comprising the amino acid sequence of SEQ ID NO: 1, 3, 5, 7, 9, or 11. Those skilled in the art can isolate proteins which are functionally equivalent to the protein comprising the amino acid sequence of SEQ ID NO: 1, 3, 5, 7, 9, or 11, by known methods (supplementary volume of "Jikken Igaku (Experimental Medicine), Idenshi Kougaku Handbook (Genetic Engineering Handbook)", pp.246-251, published by Yodosha Co., Ltd., 1991).

Examples of proteins which are functionally equivalent to the protein comprising the amino acid sequence of SEQ ID NO: 1, 3, 5, 7, 9, or 11 include proteins having bone marrow-derived cell-inducing activity.

Proteins which comprise an amino acid sequence with one or more amino acid substitutions, deletions, insertions, and/or additions in the amino acid sequence of SEQ ID NO: 1, 3, 5, 7, 9, or 11, and which are functionally equivalent to the protein comprising the amino acid sequence of SEQ ID NO: 1, 3, 5, 7, 9, or 11 include naturally-occurring proteins. Generally, eukaryotic genes have polymorphism as known in interferon genes and such. Alterations in nucleotide sequences caused by the polymorphism may result in one or more amino acid substitutions, deletions, insertions, and/or additions. Naturally-occurring proteins such as those comprising an amino acid sequence with one or more amino acid substitutions, deletions, insertions, and/or additions in the amino acid sequence of SEQ ID NO: 1, 3, 5, 7, 9, or 11, and which are functionally equivalent to the protein comprising the amino acid sequence of SEQ ID NO: 1, 3, 5, 7, 9, or 11 are included in S100A8 or S100A9 proteins of the present invention.

The present invention also includes artificially-produced mutant proteins as long as they are functionally equivalent to the protein comprising the amino acid sequence of SEQ ID NO: 1, 3, 5, 7, 9, or 11. Known methods which cause random mutations to a given nucleotide sequence include substitution(s) of base pair(s) through nitrous acid treatment of DNA (Hirose, S. et al., Proc. Natl. Acad. Sci. USA., 79: 7258-7260, 1982). This method enables random introduction of substitution(s) of base pair(s) into a specific segment by nitrous acid treatment of the segment desired to be mutated. Alternatively, technologies for site-directing a target mutation include the gapped duplex method (Kramer W. and Fritz HJ., Methods in Enzymol., 154: 350-367, 1987) and the like. A cyclic double stranded vector in which a gene to be introduced with a mutation is cloned, is separated into single strands. These single strands are hybridized with a synthetic oligonucleotide mutated at the target site. A vector-derived complementary single strand DNA linearized by a restriction enzyme is annealed with the cyclic single stranded vector, and the gap between the oligonucleotide and the vector is filled by using a DNA polymerase, which is then made into a complete double stranded vector by ligation.

The number of amino acids to be modified would be typically within 50, preferably within 30, and more preferably within 5 amino acids (for example, one amino acid).

When an amino acid is artificially substituted, substitution with an amino acid having similar properties would result in maintaining the activity of the original protein. Proteins of the present invention include proteins resulting from a conservative substitution in the above substitution of amino acid(s), and which are functionally equivalent to the protein comprising the amino acid sequence of SEQ ID NO: 1, 3, 5, 7, 9, or 11. Conservative substitution is considered important when substituting amino acid(s) of domains important for protein activities. Such a conservative substitution of amino acid(s) is well known to those skilled in the art.

Examples of amino acid groups suitable for conservative substitution include basic amino acids (such as lysine, arginine, and histidine), acidic amino acids (such as aspartic acid and glutamic acid), uncharged polar amino acids (such as glycine, asparagine, glutamine, serine, threonine, tyrosine, and cysteine), nonpolar amino acids (such as alanine, valine, leucine, isoleucine, proline, phenylalanine, methionine, and tryptophane), β branched amino acids (such as threonine, valine, and isoleucine), and aromatic amino acids (such as tyrosine, phenylalanine, tryptophane, and histidine).

Moreover, non-conservative substitution may increase protein activities (for example, constitutively activated proteins).

In addition, proteins which are functionally equivalent to the protein comprising the amino acid sequence of SEQ ID NO: 1, 3, 5, 7, 9, or 11 can be obtained by methods that utilize hybridization. That is to say, a DNA encoding S100A8 or S100A9 protein of the present invention as shown in the SEQ ID NO: 2, 4, 6, 8, 10, or 12, or a fragment thereof is used as a probe, and then DNAs that can hybridize to them are isolated. A hybridization reaction performed under stringent conditions leads to the selection of highly homologous DNA as a nucleotide sequence. This increases the chances of isolated proteins containing proteins that are functionally equivalent to the S100A8 or S100A9 protein. Examples of a highly homologous nucleotide sequence include those having 70% or more, and desirably 90% or more identity.

In a specific example, the term "stringent conditions" refers to hybridization conditions with 6x SSC, 40% formamide at 25°C and subsequent washing with 1x SSC at 55°C. The stringency depends on conditions such as salt concentration, formamide concentration, or temperature; however it is obvious for those skilled in the art to set these conditions so as to obtain necessary stringency.

With the use of hybridization, for example, DNAs encoding homologues of the S100A8 or S 100A9 proteins other than those proteins comprising the amino acid sequence of SEQ ID NO: 1, 3, 5, 7, 9, or 11 can be isolated.

Proteins which are functionally equivalent to a protein comprising the amino acid sequence of SEQ ID NO: 1, 3, 5, 7, 9, or 11 normally have a high homology with the amino acid sequence of SEQ ID NO: 1, 3, 5, 7, 9, or 11. The term "high homology" refers to a sequence identity of at least 30% or more, preferably 50% or more, more preferably 80% or more (for example, 95% or more). The identity of the nucleotide sequences and amino acid sequences can be determined using a homology search site *via* the internet [For example, homology searches such as FASTA, BLAST, PSI-BLAST, and SSEARCH can be used in the DNA Data Bank of Japan (DDBJ) [examples of which include the homology search page (Search and Analysis) at the DNA Data Bank of Japan (DDBJ) website;
http://www.ddbj.nig.ac.jp/E-mail/homology-j.html]. Furthermore, searches using BLAST can be carried out through the web site of the National Center for Biotechnology Information (NCBI) (examples of which include BLAST page at the homepage of NCBI website; http://www.ncbi.nlm.nih.gov/BLAST/; Altschul, S.F. et al., J. Mol. Biol., 1990, 215(3): 403-10; Altschul, S.F. & Gish, W., Meth. Enzymol., 1996, 266: 460-480; Altschul, S.F. et al., Nucleic Acids Res., 1997, 25: 3389-3402)].

For example, in the calculation of the identity of amino acid sequences using Advanced BLAST 2.1, the identity value (%) can be obtained by the following: blastp is used as the program, expect value is set at 10, all filters are set at OFF, BLOSUM62 is used for matrix, and gap existence cost, per residue gap cost, and lambda ratio are set at 11, 1, and 0.85, respectively (default parameters) (Karlin, S. and S. F. Altschul (1990) Proc. Natl. Acad. Sci. USA 87: 2264-68; Karlin, S. and S. F. Altschul (1993) Proc. Natl. Acad. Sci. USA 90: 5873-7).

Proteins of the present invention, or proteins functionally equivalent thereto may be proteins subjected to various modifications such as physiological modification with sugar chains and the like, labeling with fluorescence or radioactive substances, or fusion with other proteins. Particularly in recombinants that will be described later, sugar chain modification may vary depending on the hosts used for expression. However, even if there is a difference in sugar chain modifications, all proteins having properties similar to those of S 100A8 or S 100A9 proteins disclosed herein are S 100A8 or S 100A9 proteins of the present invention or proteins functionally equivalent thereto.

S 100A8 or S 100A9 proteins can be obtained not only from living materials, but also in the form of recombinants by incorporating genes that encode these proteins into an appropriate expression system. In order to obtain S100A8 or S100A9 proteins by genetic engineering techniques, the above-mentioned DNAs which encode S100A8 or S100A9 proteins may be incorporated into an appropriate expression system, and they can then be expressed. Examples of host/vector systems applicable to the present invention include the expression vector pGEX and *E. coli.* With pGEX, foreign genes can be expressed as a fusion protein with glutathione-S-transferase (GST) (Gene, 67: 31-40, 1988). pGEX incorporated with a gene encoding the S 100A8 or S 100A9 protein is introduced into *an E. coli* strain such as BL21 by heat shock, incubated for an appropriate time and then isopropylthio-β-D-galactoside (IPTG) is added to induce the expression of GST-fused S100A8 or GST-fused S100A9 proteins. Since GST of the present invention adsorbs onto Glutathione Sepharose 4B, the expression product is readily separated and purified by affinity column chromatography.

In addition, the following may also be applied as host/vector systems to obtain recombinants of S100A8 or S100A9 proteins. First, when bacteria are used as hosts, expression vectors for fusion proteins that utilize histidine-tag, HA-tag, FLAG-tag, and the like are commercially available. Regarding yeasts, yeasts belonging to the genus *Pichia* are known to be effective for the expression of sugar chain-containing proteins. In terms of the addition of sugar chains, expression systems that utilize baculovirus vector with insect cells as a host are also useful (Bio/Technology, 6: 47-55, 1988). Further, using mammalian cells, transfection of a vector is carried out using promoters such as CMV, RSV, and SV40. Any of these host/vector systems can be used as an expression system of S 100A8 or S 100A9 proteins. Moreover, genes can also be introduced using viral vectors such as retrovirus vectors, adenovirus vectors, and adeno-associated virus vectors.

Thus obtained proteins of the present invention may be isolated intracellularly or extracellularly (medium and such), and can be purified as proteins that are substantially pure and homogenous. Proteins may be separated and purified using separation and purification methods which are commonly used in protein purification, and are not particularly limited. For example, proteins can be separated and purified by appropriately selecting and combining a chromatography columns, filters, ultrafiltration, salting out, solvent precipitation, solvent extraction, distillation, immunoprecipitation, SDS-polyacrylamide gel electrophoresis, isoelectric focusing electrophoresis, dialysis, recrystallization, and the like.

Examples of chromatographies include affinity chromatography, ion-exchange chromatography, hydrophobic chromatography, gel filtration, reverse phase chromatography, and adsorption chromatography (Marshak et al., Strategies for Protein Purification and Characterization: A Laboratory Course Manual. Ed Daniel R. Cold Spring Harbor Laboratory Press, 1996). These chromatographies can be performed using liquid phase chromatographies such as HPLC and FPLC.

Moreover, proteins of the present invention are preferably substantially purified proteins. Here, the term "substantially purified" means that the protein purity of the present invention (proportion of the protein of the present invention in total protein components) is 50% or more, 60% or more, 70% or more, 80% or more, 90% or more, 95% or more, 100% or close to 100%. The upper limit for "close to 100%" depends on the purification techniques and analytical techniques of those skilled in the art, of which examples are 99.999%, 99.99%, 99.9%, 99%, and the like.

Moreover, a substantially purified protein includes any protein purified by any purification method as long as the protein purity is as mentioned above. Examples include, but are not limited to, proteins substantially purified by appropriately selecting and combining the above-mentioned chromatography columns, filters, ultrafiltration, salting out, solvent precipitation, solvent extraction, distillation, immunoprecipitation, SDS-polyacrylamide gel electrophoresis, isoelectric focusing electrophoresis, dialysis, recrystallization, and the like.

Cells where S 100A8 or S 100A9 proteins of the inducing agents or tissue regeneration-promoting agents of the present invention are released or secreted basically include all types of tissue-derived cells *in vivo.* Cells which can be readily collected and cultured are exemplified by, but are not limited to, fibroblasts (such as normal skin fibroblasts and cell lines derived therefrom). Moreover, cells secreting S100A8 or S100A9 proteins can also be produced by the following manner. A vector is produced by inserting an S100A8 or S100A9 protein-encoding DNA, or an S 100A8 or S 100A9 protein-encoding DNA linked with a secretion signal-encoding DNA (ATG CAG ACA GAC ACA CTC CTG CTA TGG GTA CTG CTG CTG TGG GTT CCA GGT TCC ACT GGT GAC; SEQ ID NO: 15), into a known expression vector or a gene therapy vector. The produced vector is introduced into mammalian cells such as fibroblasts (such as normal skin fibroblasts and cell lines derived therefrom), insect cells, and other cells. Examples of secretion signal-encoding DNAs include, but are not limited to, DNAs with the above-described sequences. There are no particular limitations in the animal type from which these cells derive, although cells from the animal type of the target animal subjected to tissue regeneration, cells from the target itself, or cells derived from a blood relative of the target subjected to tissue regeneration are preferably used.

DNAs which encode S 100A8 or S 100A9 proteins of the inducing agents or tissue regeneration-promoting agents of the present invention may be cDNAs, genomic DNAs, natural DNAs, or artificially-synthesized DNAs as long as they encode the S 100A8 or S 100A9 protein. DNAs which encode S 100A8 or S 100A9 proteins are normally contained in the inducing agents or tissue regeneration-promoting agents of the present invention in a form inserted in vectors (such as gene therapy vectors).

Examples of the gene therapy vectors of the present invention include, but are not limited to, plasmid vectors, retrovirus vectors, lentivirus vectors, adenovirus vectors, adeno-associated virus vectors, Sendai virus vectors, Sendai virus envelope vectors, and papilloma virus vectors. The gene therapy vectors may contain promoter DNA sequences which effectively induce gene expression, factors that regulate gene expression, and molecules which are necessary for maintaining DNA stability.

Inducing agents and tissue regeneration-promoting agents of the present invention may also contain: partial peptides of S 100A8 or S 100A9 protein which have an activity of inducing bone marrow-derived cell; cells secreting these partial peptides; or vectors inserted with the DNAs encoding these partial peptides.

Methods for administering inducing agents or tissue regeneration-promoting agents of the present invention include oral parenteral administrations. Specific examples of the administration methods include administration by injection, nasal administration, lung administration, dermal administration. For example, inducing agents or tissue regeneration-promoting agents of the present invention can be administered by intravascular injection (intraarterial injection, intravenous injection, or such), intramuscular injection, intraperitoneal administration, subcutaneous injection, or such. Alternatively, they can be locally administered (for example, under the skin, intradermally, skin surface, eye balls or palpebral conjunctiva, mucous membrane of the nasal cavity, in the oral cavity, mucous membrane of the digestive tract, mucous membrane of he vagina or uterus, damaged sites, or such).

The method of administration may be appropriately selected according to the age and the symptoms of the patient. When an S100A8 or S100A9 protein is administered, the dose of the protein per use can be selected within a range of 0.0000001 mg to 1,000 mg per kg body weight of a patient. Alternatively, the dose can be selected within a range of 0.00001 mg to 100,000 mg per body of patient, for example. When administering cells secreting S100A8 or S100A9 proteins or gene therapy vectors inserted with DNAs encoding S100A8 or S100A9 proteins they may be administered such that the amounts of S 100A8 or S 100A9 protein in the damaged tissue are within the above range. However, the dosage of the inducing agents or tissue regeneration-promoting agents of the present invention is not limited thereto.

Inducing agents or tissue regeneration-promoting agents of the present invention can be formulated according to the usual methods (for example, Remington's Pharmaceutical Science, latest edition, Mark Publishing Company, Easton, U.S.A), and may contain pharmaceutically acceptable carriers and additives together. Examples include surfactants, excipients, colorants, perfumes, preservatives, stabilizers, buffers, suspending agents, isotonizing agents, binders, disintegrants, lubricants, flow promoters, and flavoring agents, although they are not limited thereto and other common carriers may be appropriately used. Specific examples include light anhydrous silicic acid, lactose, crystalline cellulose, mannitol, starch, carmellose calcium, carmellose sodium, hydroxypropylcellulose, hydroxypropylmethylcellulose, polyvinylacetaldiethylamino acetate, polyvinylpyrrolidone, gelatin, medium-chain fatty acid triglyceride, polyoxyethylene hydrogenated castor oil 60, white sugar, carboxymethyl cellulose, corn starch, and inorganic salts.

Moreover, applications of the above-mentioned S 100A8 or S 100A9 proteins, cells secreting S100A8 or S100A9 proteins, vectors inserted with DNAs encoding S100A8 or S100A9 proteins, partial peptides of S 100A8 or S 100A9 proteins, cells secreting these partial peptides; or vectors inserted with DNAs encoding these partial peptides can be expressed as (1) to (5) below:
(1) methods for promoting a damaged tissue regeneration, which comprise the step of administering to a subject whose tissues are damaged, S 100A8 or S 100A9 proteins, cells that secrete the proteins, vectors inserted with DNAs encoding the proteins, partial peptides of the proteins, cells that secrete the partial peptides, or vectors inserted with DNAs encoding the partial peptides;
(2) methods for inducing bone marrow cells to damaged sites, which comprise the step of administering to a subject whose tissues are damaged, S 100A8 or S 100A9 proteins, cells that secrete the proteins, vectors inserted with DNAs encoding the proteins, partial peptides of the proteins, cells that secrete the partial peptides, or vectors inserted with DNAs encoding the partial peptides;
(3) uses of S 100A8 or S 100A9 proteins, cells that secrete the proteins, vectors inserted with DNAs encoding the proteins, partial peptides of the proteins, cells that secrete the partial peptides, or vectors inserted with DNAs encoding the partial peptides, in the preparation of agents for inducing bone marrow or agents for promoting tissue regeneration;
(4) S 100A8 or S 100A9 proteins, cells that secrete the proteins, vectors inserted with DNAs encoding the proteins, partial peptides of the proteins, cells that secrete the partial peptides, or vectors inserted with DNAs encoding the partial peptides, for use in methods for inducing bone marrow cells to damaged tissues; and
(5) S 100A8 or S 100A9 proteins, cells that secrete the proteins, vectors inserted with DNAs encoding the proteins, partial peptides of the proteins, cells that secrete the partial peptides, and vectors inserted with DNAs encoding the partial peptides, for use in methods for promoting tissue regeneration of damaged tissues.

Subjects in which the above-described tissues are damaged include humans and non-human animals, for example, humans, mice, rats, monkeys, pigs, dogs, rabbits, hamsters, and guinea pigs, but are not limited thereto.

All prior art documents cited herein are incorporated by reference herein.

### Examples

Hereinbelow, the present invention will be specifically described with reference to the Examples, but it is not to be construed as being limited thereto.

### [Example 1]

Purpose: To assess contribution of bone marrow-derived cells to the functional regeneration *of in vivo* grafted skin tissue

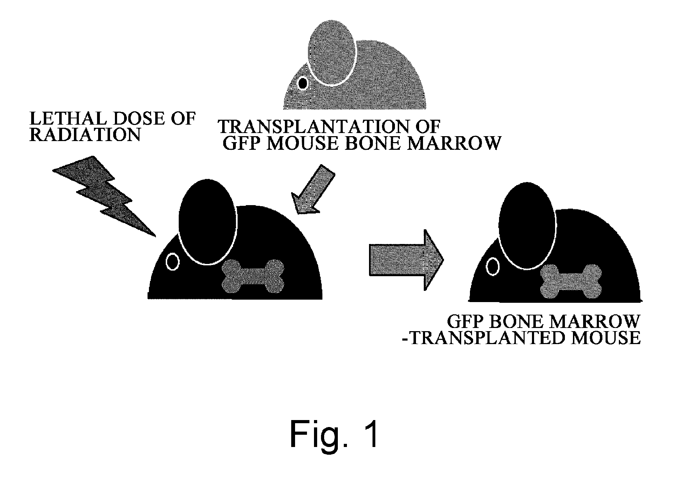

Methods: Studies were conducted to achieve the above purpose.
(1) The degree at which bone marrow-derived cells contribute to the functional regeneration of grafted skin was assessed using a system *of in vivo* skin grafting in GFP bone marrow-transplanted mice. Specifically, male C57BL/6 mice (six to eight weeks old) were irradiated at a lethal dose (10 Gy), and green fluorescent protein (GFP) transgenic mouse-derived bone marrow cells (5 x 10⁶ cells/0.1 ml of physiological phosphate buffered saline, pH 7.4) were transplanted into the mice *via* the caudal vein immediately after the irradiation (Fig. 1).
(2) After the engraftment of transplanted bone marrow cells (six weeks) was confirmed, neonatal mouse (female) skin was transplanted to the dorsal skin of the resulting GFP bone marrow-transplanted mice.
(3) After confirming the engraftment of grafted skin and sufficient skin tissue regeneration (four weeks), the degree of GFP fluorescence accumulation in the area of grafted skin was observed under a fluorescence stereomicroscope.
(4) The grafted skin was obtained by biopsy under inhalation anesthesia. Skin cryosections (6 µm) were prepared using a microtome with cooling apparatus, and fixed for 30 minutes with 4% paraformaldehyde. Then, cell nuclei in the tissues were stained with DAPI. After mounting the tissue using a mounting medium containing anti-fluorescence photobleaching agent, the tissues were observed under a confocal laser microscope to assess the presence of GFP-positive bone marrow-derived cells.

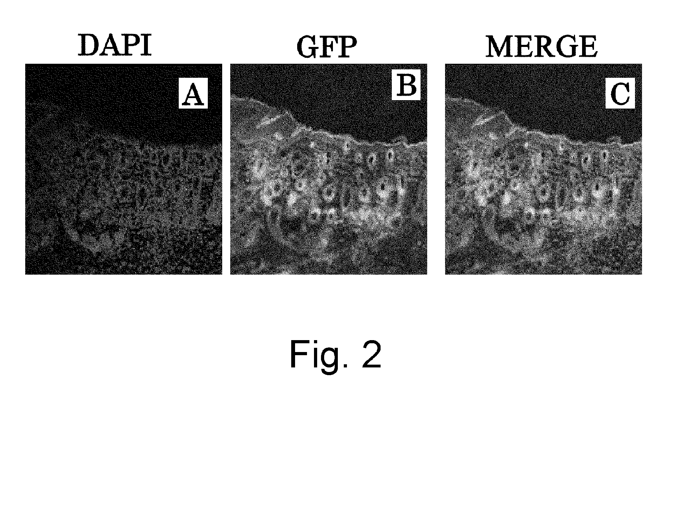

Results: In the system *of in vivo* skin grafting in GFP bone marrow-transplanted mice, GFP fluorescence was observed in the majority of epidermal keratinocytes and dermal fibroblasts as well as smooth muscle cells and adipocytes of the regenerated skin tissues, suggesting that these cells were derived from the bone marrow (Fig. 2). Specifically, bone marrow-derived stem cells served as a source for most of the epithelial cells and mesenchymal cells required for the functional regeneration of the grafted skins.

Discussion: The results described above suggest that upon skin damage, bone marrow cells accumulate at the damaged site and differentiate into various types of organs constituting the skin, thereby contributing to functional regeneration of the skin. Meanwhile, it is speculated that the grafted skin contains substances that attract bone marrow cells which are capable of differentiating into various types of organs.

It has been reported that bone marrow contains two types of stem cell systems: hematopoietic stem cells and mesenchymal stem cells. It would be difficult to anticipate that a large number of bone marrow-derived epithelial cells and mesenchymal cells mobilized into the grafted skin are provided by bone marrow-derived hematopoietic stem cells as shown by the present research. This strongly suggests the possibility that bone marrow-derived mesenchymal stem cells contribute to the functional regeneration of grafted tissues. Specifically, it is anticipated that immediately after skin grafting, factors that mobilize bone marrow-derived mesenchymal stem cells are released from the grafted skin in a state of hemostasis/necrosis, and mobilize mesenchymal stem cells to the grafted skin from bone marrow *via* the peripheral blood circulation, thereby inducing functional regeneration of the skin tissue.

### [Example 2]

Purpose: To identify bone marrow-derived tissue stem cell-inducing factors in skin tissue extracts

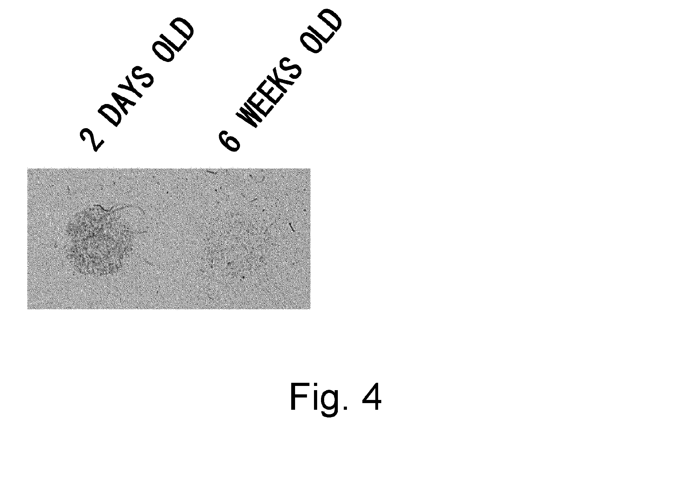

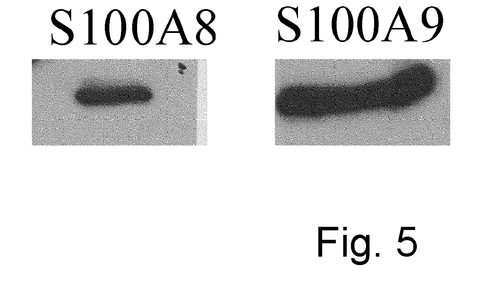

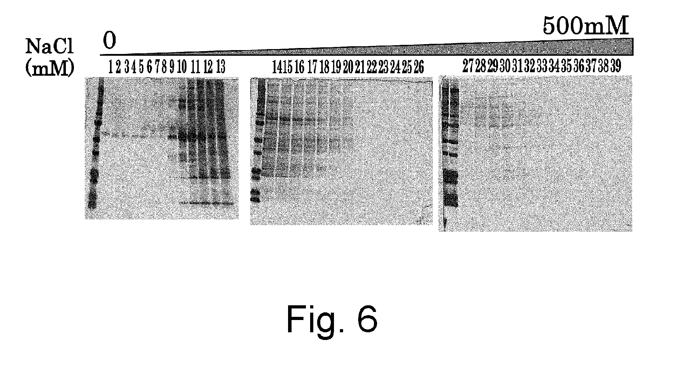

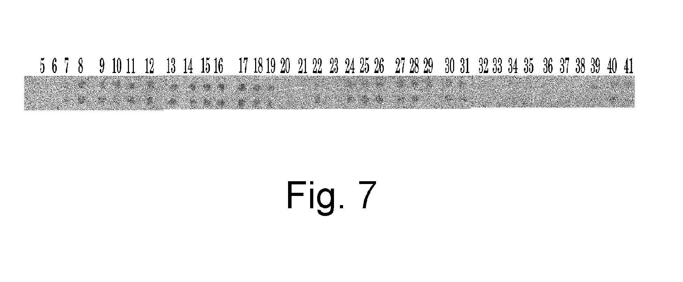

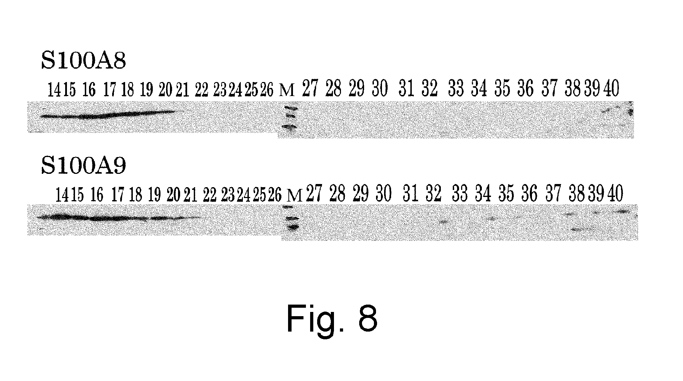

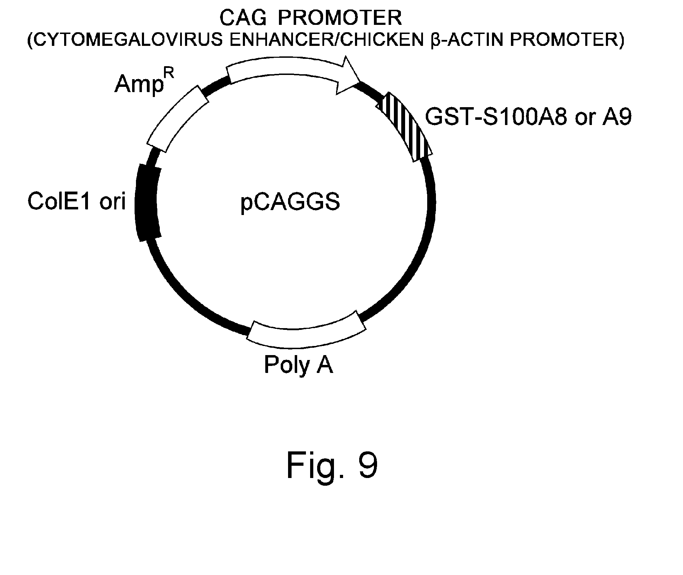

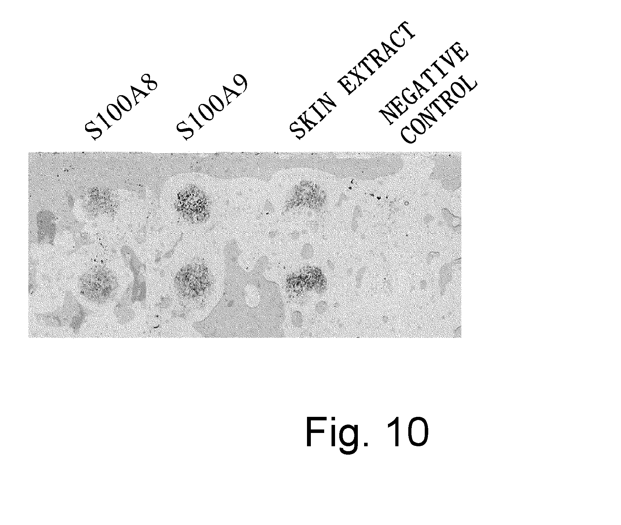

Methods: By the method described below, study was conducted to identify factors responsible for mobilizing bone marrow mesenchymal stem cells, which were predicted to be released from excised skin under hemostatic condition.
(1) Bone marrow cells were harvested from the thighbones or crural bones of C57BL/6 mice to obtain mouse bone marrow-derived mesenchymal stem cells. The cells were seeded into a cell culture dish with D-MEM (Nacalai) supplemented with 10% fetal bovine serum as a culture medium and cultured at 37°C under 5% carbon dioxide gas. When the cells were grown to occupy an area of 70 to 100% relative to the bottom of the culture dish, the cells were detached from the culture dish using 0.25% trypsin/1 mM EDTA (Nacalai). The cells were then passaged under the same culture conditions. After at least five passages, the adherent cells were isolated and further cultured, and analyzed for cell surface antigens by flow cytometry. The result showed that the cells were positive for CD44 and Sca-1, and negative for Lin, CD45, and c-kit. It was confirmed that the cells can differentiate into osteocytes and adipocytes and thus have the characteristics of bone marrow mesenchymal stem cells.
(2) Free skin pieces isolated from five heads of neonatal mice (two-day-old) were immersed in 5 ml of physiological phosphate buffered saline (PBS, pH 7.4). After 24 hours of incubation at 4°C, the sample was centrifuged at 440 G at 4°C for ten minutes to remove tissues. The supernatant was collected as skin extract. In addition, in the same way, free skin pieces isolated from a six-week-old mouse were immersed in 5 ml of physiological phosphate buffered saline (PBS, pH 7.4). After incubation at 4°C for 24 hours, the samples were centrifuged at 440 G at 4°C for ten minutes to remove tissues. The supernatants were collected as skin extract.
(3) To confirm whether the prepared skin extract has the activity of inducing bone marrow mesenchymal stem cells, the present inventors used the Boyden chamber to examine the chemotactic activity for previously cloned bone marrow-derived mesenchymal cells derived from C57BL6 mice. Specifically, a mixture of DMEM (20 µl) and skin extract (5 µl) from two-day-old or six-week-old mice was added into the bottom compartment (a volume of 25 µl) of a Boyden chamber, and a polycarbonate membrane with 8-µm micropores was placed on top. Then, the upper compartment (a volume of 50 µl) of the Boyden chamber was placed in contact with the membrane, and a suspension of bone marrow-derived mesenchymal stem cells (5 x 10⁴ cells/50 ml of culture medium (DMEM supplemented with 10% fetal bovine serum)) was added to the upper compartment. The chamber was incubated in a CO₂ incubator at 37°C for four to 24 hours. After incubation, the upper unit of the chamber was removed. The thin silicone film was detached and the number of bone marrow-derived mesenchymal stem cells migrating into the bottom compartment through the micropores was quantitatively determined by staining the cells (Fig. 4).
(4) About 2-cm² skin specimens were excised from two-day-old and six-week-old mice and immediately frozen in liquid nitrogen. The skin specimens were crushed in a mortar. RNAs were extracted and purified from the samples using RNeasy (Qiagen). Using the purified RNAs, microarray assay was carried out to screen for mRNA expressed at higher levels in the two-day-old mice. 767 genes showed two or more times greater scores in the two-day-old mice. Of these genes, proteins with high affinity for heparin, potential secretory proteins, and genes whose scores were six or more times greater in the two-day-old mice were examined and S100A9 was found as the 57^{th} gene from the top. Thus, S100A9 and S100A8, which is known to form a heterodimer with S100A9, in the skin extract from the two-day-old mice was detected by Western blotting. Specifically, 5 µl of the skin extract from the two-day-old mice was combined with 5 µl of SDS-PAGE sample buffer (Bio-Rad). The mixture was heated in a heat block at 98°C for five minutes, and then cooled to 25°C. The resulting sample was applied onto 12.5% acrylamide gel e-PAGEL (ATTO) and electrophoresed at 40 mA for 75 minutes using an electrophoretic device (ATTO). The gel was collected after electrophoresis. Using a blotting device (ATTO), proteins in the gel were transferred to PVDF membrane (7 cm by 9 cm, Millipore) pretreated with 100% methanol. After 75 minutes of protein transfer at 120 mA, the PVDF membrane was removed and shaken at room temperature for 30 minutes in PBS (Nacalai) containing 4% skim milk. Then, the removed PVDF membrane was soaked in 5 µl of anti-S100A8 antibody (R&D) or anti-S100A9 antibody (R&D) each diluted with 10 ml of PBS containing 4% skim milk, and shaken at room temperature for 60 minutes. After the antibody solution was removed, the membrane was shaken in 30 ml of PBS containing 0.1% Tween20 at room temperature for five minutes. This washing was repeated five times. Then, the membrane was soaked in 5 µl of HRP-labeled anti-goat IgG antibody (GE healthcare) diluted with 10 ml of PBS containing 4% skim milk, and shaken at room temperature for 45 minutes. After the antibody solution was removed, the membrane was washed with 30 ml of PBS containing 0.1% Tween20 at room temperature for five minutes while shaking. This washing was repeated five times. The membrane was treated for luminescence using ECL Detection Kit (GE healthcare), and then exposed on a film. Signals for S100A8 and S100A9 proteins were gained by developing the film in a developing apparatus (Fig. 5).
(5) Factors having the activity of mobilizing bone marrow-derived mesenchymal stem cells in skin extracts were purified by heparin affinity column chromatography. The experiment described below was carried out using an FPLC device (GE healthcare). First, the skin extract of two-day-old mice was diluted 10-fold with nine volumes of 20 mM phosphate buffer (pH 7.5) at 4°C (dilution solution A). 300 ml of 20 mM phosphate buffer (pH 7.5) was run through a HiPrep 16/10 Heparin FF (GE Healthcare) column to equilibrate the column in advance, and dilution solution A was loaded onto the column. Then, the column was washed with 300 ml of 20 mM phosphate buffer (pH 7.5). 20 mM phosphate buffer (pH 7.5) containing 10 mM NaCl (solution A) and 20 mM phosphate buffer (pH 7.5) containing 500 mM NaCl (solution B) were prepared to elute the adsorbed protein. Elution was started with [100% solution A + 0% solution B], and then the proportion of solution B was gradually increased. Finally, the column was eluted with [0% solution A + 100% solution B]. The total elution volume was 150 ml. The eluate was fractionated into silicone-coated tubes (3 ml/tube). 5 µl each of the fractionated samples were mixed with 5 µl of SDS-PAGE sample buffer (Bio-Rad). The mixtures were heated in a heat block at 98°C for five minutes, and then cooled to 25°C. The samples were applied onto an acrylamide gel e-PAGEL (5-20% gradient, ATTO), and electrophoresed at 40 mA for 75 minutes using an electrophoresis device. After the electrophoresis, the electrophoresed protein was detected using the Dodeca Silver Stain Kit (Bio-Rad) (Fig. 6).
   The chemotactic activity of fractionated samples was assayed in the same way as described above using a Boyden chamber (Fig. 7).
   The presence of S 100A8 and S 100A9 proteins in the fractionated samples was detected in the same way as described above by Western blotting (Fig. 8).
(6) RNA was extracted from neonatal mouse skin using Trizol (Invitrogen), and then cDNA was synthesized from the RNA using the SuperScript III cDNA Synthesis Kit (Invitrogen). cDNAs of S 100A8 and S 100A9 were amplified by the polymerase chain reaction (PCR) method using the cDNA as a template. These cDNAs were each inserted into a mammalian cell protein-expression plasmid vector, pCAGGS, to express the proteins in which a GST-tag sequence (amino acid sequence/SEQ ID NO: 13; DNA sequence/SEQ ID NO: 14) is attached to the N-terminus of their amino acid sequences (Fig. 9). pCAGGS-GST-S100A8 or pCAGGS-GST-S100A9 were each transfected into a human fetal kidney cell-derived cultured cell line HEK293 using a lipofection reagent (Invitrogen). 48 hours after transfection, the cells and culture supernatant were collected, and centrifuged at 4,400 G at 4°C for five minutes. The supernatant (Supernatant A) and cells were collected separately. PBS containing 0.1% Tween20 was added to the cells, and the suspension was sonicated on ice for 30 seconds to disrupt the cell membrane. After centrifugation at 4,400 x g at 4°C for five minutes, the resulting supernatant was collected (Supernatant B). Supernatants A and B were combined together and loaded onto a HiTrap GST FF column (5 ml; GE Healthcare) whose buffer had been replaced with 30 ml of PBS in advance. After loading, the column was washed with 100 ml of PBS, and the adsorbed protein was eluted with 20 mM phosphate buffer (pH 8) containing reduced glutathione. The chemotactic activity of recombinant S100A8 and S100A9 for bone marrow mesenchymal stem cells was assessed using the Boyden chamber. The samples were prepared by dissolving purified S 100A8 or S 100A9 protein at 0.1 ng/µl in DMEM, or by diluting the skin extract of two-day-old mice with four volumes of DMEM, and added into the bottom compartment of the Boyden chamber. A negative control prepared as follows was used the same way: protein was extracted from cells transfected with a control vector which does not carry the cDNA of S 100A8 or S 100A9 as an insert; and then a fraction was eluted from a HiTrap GST FF column. After a sample was added into the bottom compartment, a polycarbonate membrane with 8-µm micropores was placed on top. Then, the upper unit (a volume of 50 µl) of Boyden chamber was placed in contact with the membrane, and a suspension of bone marrow-derived mesenchymal stem cells (5 x 10⁴ cells/50 ml of culture medium (DMEM supplemented with 10% fetal bovine serum)) was added to the upper chamber. The chamber was incubated in a CO₂ incubator at 37°C for four to 24 hours. After incubation, the upper unit of the chamber was removed. The polycarbonate membrane was detached and the number of bone marrow-derived mesenchymal stem cells migrating into the bottom compartment through the micropores was quantitatively determined by staining the cells (Fig. 10).
(7) Eight-week-old male mice were injected with 250 µl of the above-described purified GST-S100A8 or S100A9 recombinant proteins (1ng/µl) *via* the caudal vein. 12 hours after injection 1 ml of peripheral blood was collected from the hearts of the mice under inhalation anesthesia with isoflurane using a 1-ml heparin-coated syringe. The blood samples were each combined with 3 ml of PBS, and then gently overlaid onto 3 ml of Ficoll (GE healthcare). The resulting samples were centrifuged using centrifuge at 400 x g at 25°C for 40 minutes. The cells in the opaque middle layer were collected as a mononuclear cell fraction. 1 ml ofHLB solution (Immuno-Biological Laboratories Co., Ltd.), a hemolytic agent, was added to the collected cells, and the cells were incubated at room temperature for five minutes. This hemolytic treatment was repeated twice. After adding 10 ml of PBS, the cells were centrifuged at 440 x g at 25°C for five minutes. The resulting supernatants were removed, and the cells were collected. 1,000,000 cells were incubated at room temperature for 20 minutes with a PE-labeled anti-mouse PDGFRα antibody (e-Bioscience), PE-labeled anti-mouse PDGFRβ antibody (e-Bioscience), FITC-labeled anti-mouse CD45 antibody (BD biosciences), and PerCy5-labeled anti-mouse CD44 antibody (BD biosciences), each diluted 100-fold with PBS. Then, the cells were centrifuged at 440 x g at 25°C for five minutes. The supernatants were removed. 400 µl of PBS containing 1% paraformaldehyde was added to the cells to prepare samples for flow cytometric analysis. Antibodies were used in the following combinations:
   (I) PDGFRα/CD45/CD44
   (II) PDGFβ/CD45/CD44

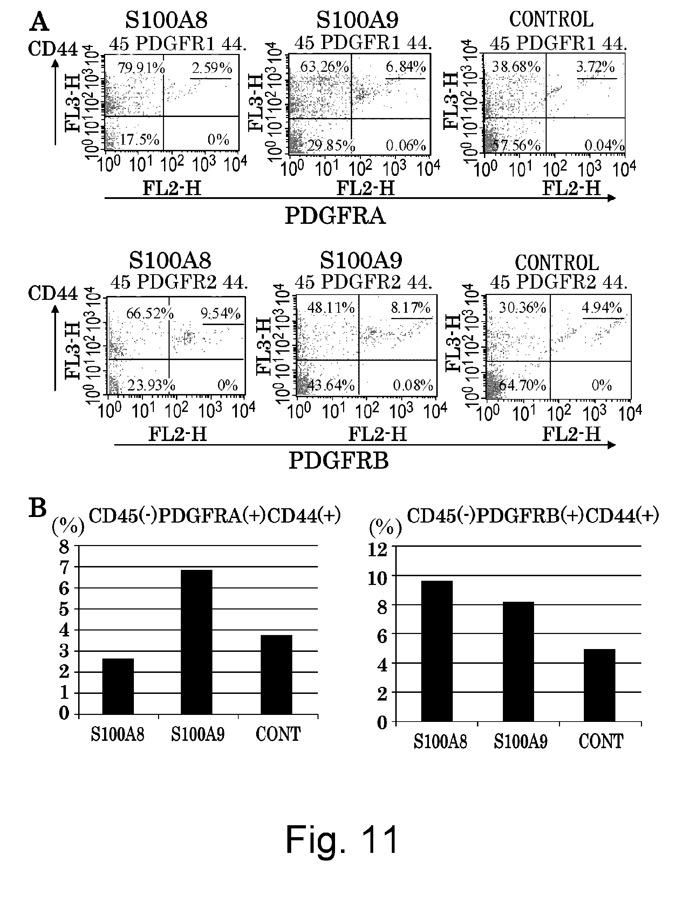

The ratio of cells expressing PDGFRα (or β) and CD44 to cells that were weakly positive or negative for CD45 was determined based on the analysis result (Figs. 11A and B).

Results: Skin samples excised from two-day-old and six-week-old mice were assessed for the activity of mobilizing bone marrow mesenchymal stem cells. The activity of skin extract from two-day-old mice was demonstrated to be stronger than that of the skin extract from six-week-old mouse. Strong S100A9 expression in the skin from two-day-old mice was found by DNA microarray analysis. Crude samples of skin extracts purified on a heparin column exhibited correlation between the migrating activity of mesenchymal stem cells and the contents of S100A9 and S100A8. Expression vectors for these proteins were constructed, and the recombinant proteins were produced using HEK293 and purified. The migrating activity of bone marrow mesenchymal stem cells was confirmed in the purified S 100A8 and S 100A9 samples by assays using Boyden chamber. Furthermore, when intravenously administered to mice, the proteins also exhibited the activity of mobilizing a population of PDGFRα and CD44 double-positive cells to peripheral blood (Fig. 11).

Discussion: The present inventors for the first time in the world discovered in the present invention that free skin pieces produce S 100A8 and S 1 00A9, and the produced S 100A8 and S 100A9 proteins had strong activities of mobilizing bone marrow-derived mesenchymal stem cells. Meanwhile, bone marrow mesenchymal stem cells are known as pluripotent stem cells that differentiate into bone tissues, adipose tissues, cartilage tissues, fibroblasts, and the like. Recently, it has been indicated that bone marrow-derived cells also include pluripotent stem cells that differentiate into tissues such as cardiac muscle, nerve cells, and epidermal cells. Since the present invention demonstrates that the epidermal cells, hair follicle cells, fibroblasts of subcutaneous tissues, and such in the grafted skin are constituted by bone marrow-derived cells, S 100A8 and S 100A9 can be speculated to be responsible for mobilizing bone marrow-derived tissue stem cells to the skin graft to induce functional repair of damaged tissues. Even by intravenous injection, S100A8 and S100A9 can mobilize bone marrow mesenchymal stem cells to peripheral blood. Thus, S 100A8 and S 100A9 can also be administered *via* peripheral circulation to tissues located deep inside the body where local administration is difficult (brain, heart, spinal cord, etc.). The present inventors believe that effects such as shortening the healing time, functional regeneration of damaged tissues, and such can be expected in the healing process for not only damaged skin tissues but also various damaged tissues such as brain, muscle, and bone by using the present invention in pharmaceuticals, which enables local mobilization of the bone marrow-derived tissue stem cells including mesenchymal stem cells in regeneration of damaged tissues.

### [Example 3]

Purpose: To assess the therapeutic effect of S 100A8 on cutaneous ulcer in normal and diabetic mice

Methods: Recombinant S 100A8 protein was administered to cutaneous ulcer model mice to assess its therapeutic effect on ulcer. Test mice used were: C57/B16 mice transplanted with bone marrow cells expressing GFP, and BKS.Cg-m+/+Leprdb/J (db mice), which are diabetes model mice. Cutaneous ulcers with a diameter of 6 mm were formed on the skin of the mice. When cutaneous ulcer is formed in mice, the surrounding skin close to the skin defect rapidly shrinks. In this experiment, to create a therapeutic model for skin defect, in which skin defect is treated not through shrinkage but by covering it with regenerated skin, a silicone rubber disc with an outer diameter of 10 mm, inner diameter of 6 mm, and thickness of 0.5 mm was fixed at the skin defect site to the skin surrounding the ulcer using an adhesive agent for skin surgery (Aron alpha A) and nylon suture to prepare a model for treating skin defect by covering it with regenerated skin, not by shrinkage of the skin. Then, the recombinant S100A8 protein was directly administered to the ulcer surface at 1.5 µg/day every day for seven days. Furthermore, the ulcer surface was protected with film dressing Tegaderm (3M) to prevent desiccation of the ulcer surface. The ulcer surface area was measured over time to assess the therapeutic effect.

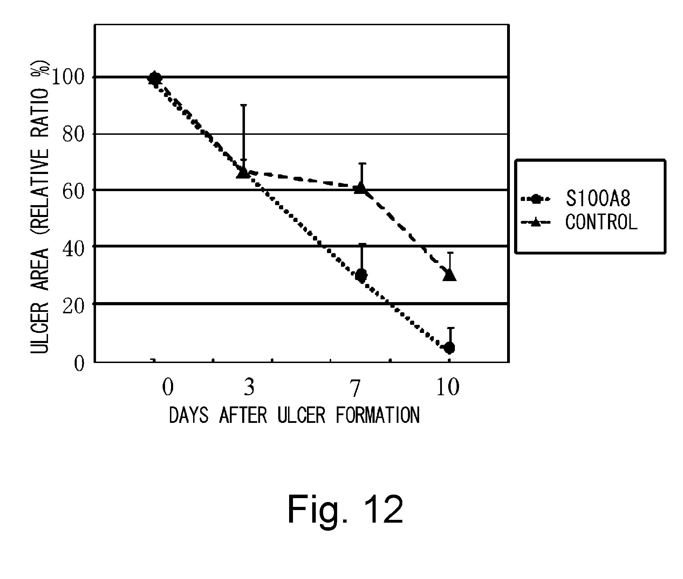

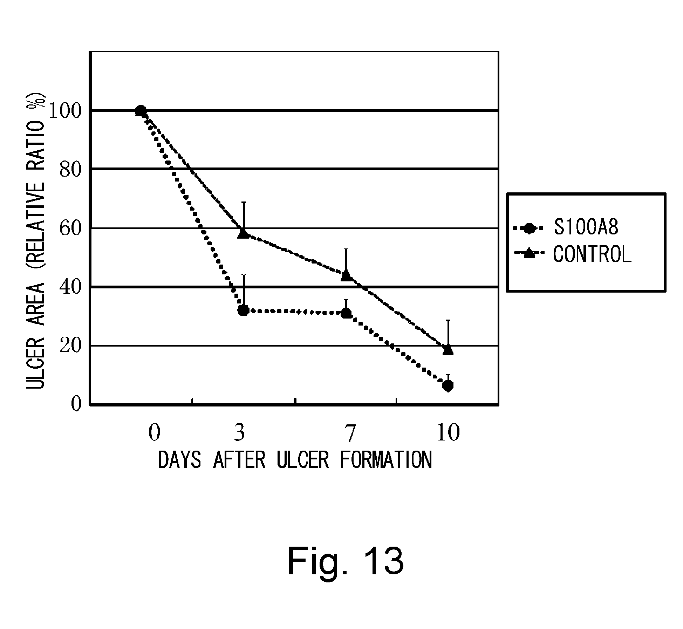

Results and discussion: In normal mice, the ulcer surface area was significantly reduced in the S100A8-treated group seven days after the start of treatment as compared to the control group (Fig. 12). Furthermore, in diabetes mice, the ulcer surface area was also significantly reduced in the S 100A8-treated group seven days after the start of treatment as compared to the control group (Fig. 13). In other words, the significant cutaneous ulcer-reducing effect was observed in both normal and diabetes mice. From this result, it is confirmed that S100A8 has the therapeutic effect on cutaneous ulcer in not only normal mice but also diabetes mice.

### Industrial Applicability

The present invention provides bone marrow-derived cell-inducing agents and tissue regeneration-promoting agents that comprise S100A8 or S100A9. The development of S100A8 or S 100A9 as pharmaceuticals for mobilizing bone marrow-derived cells enables novel regeneration medicine that promotes functional regeneration of intractable damaged tissues. S 100A8 and S 100A9 are released from cells of tissues that are in a state of necrosis under hypoxic condition due to reduction of blood flow, and exert the effect of mobilizing to the local site bone marrow-derived cells or various cells derived from these cells. The mobilized bone marrow-derived cells differentiate into cell lineages required for each tissue type to promote the recovery of lost functions due to hemostasis/necrosis (the so-called functional tissue regeneration). For example, bone marrow-derived cells are mobilized to the site of skin ulcer by administering S 100A8 or S 100A9 to the intractable skin sites caused by blood flow obstruction of the skin. The local blood flow will be improved when the mobilized cells differentiate into vascular endothelial cells. Furthermore, instead of fibrous healing, functional skin regeneration with essential dermal appendages is induced and promoted when the mobilized cells differentiate into vascular endothelial cells, nerve cells, hair follicle cells, and even epidermal cells. In addition, it is expected that S100A8 and S100A9 are also effective as a functional tissue regeneration-inducing agent in necrotic conditions of other organs due to hemostasis, such as myocardial infarction and brain infarction.

## Claims

1. A bone marrow cell-inducing agent, which comprises a component of any of (a) to (f) described below:
(a) an S100A8 protein;
(b) a cell that secretes an S 100A8 protein;
(c) a vector inserted with a DNA encoding an S 100A8 protein;
(d) an S 100A9 protein;
(e) a cell that secretes an S 100A9 protein; and
(f) a cell that secretes an S 100A9 protein.

2. A tissue regeneration-promoting agent, which comprises the component of any of (a) to (f) described below:
(a) an S100A8 protein;
(b) a cell that secretes an S 100A8 protein;
(c) a vector inserted with a DNA encoding an S 100A8 protein;
(d) an S 100A9 protein;
(e) a cell that secretes an S 100A9 protein; and
(f) a cell that secretes an S 100A9 protein.


SEQUENCE LISTING

<110> GENOMIX CO., LTD.
      OSAKA UNIVERSITY

<120> Pharmaceutical agent for promoting the functional regeneration of damaged
tissue

<130> G6-A0803P

<150> JP 2008-119324
<151> 2008-04-30

<160> 15

<170> PatentIn version 3.4

<210> 1
<211> 89
<212> PRT
<213> Mus musculus

<400> 1

Met Pro Ser Glu Leu Glu Lys Ala Leu Ser Asn Leu Ile Asp Val Tyr
1               5                   10                  15

His Asn Tyr Ser Asn Ile Gln Gly Asn His His Ala Leu Tyr Lys Asn
            20                  25                  30

Asp Phe Lys Lys Met Val Thr Thr Glu Cys Pro Gln Phe Val Gln Asn
        35                  40                  45

Ile Asn Ile Glu Asn Leu Phe Arg Glu Leu Asp Ile Asn Ser Asp Asn
    50                  55                  60

Ala Ile Asn Phe Glu Glu Phe Leu Ala Met Val Ile Lys Val Gly Val
65                  70                  75                  80

Ala Ser His Lys Asp Ser His Lys Glu
                85

<210>  2
<211>  270
<212>  DNA
<213>  Mus musculus

<400>  2
atgccgtctg aactggagaa ggccttgagc aacctcattg atgtctacca caattattcc      60

aatatacaag gaaatcacca tgccctctac aagaatgact tcaagaaaat ggtcactact     120

gagtgtcctc agtttgtgca gaatataaat atcgaaaact tgttcagaga attggacatc     180

aatagtgaca atgcaattaa cttcgaggag ttccttgcga tggtgataaa agtgggtgtg     240

gcatctcaca aagacagcca caaggagtag                                      270

<210>  3
<211>  89
<212>  PRT
<213>  Rattus norvegicus

<400>  3

Met Ala Thr Glu Leu Glu Lys Ala Leu Ser Asn Val Ile Glu Val Tyr
1               5                   10                  15

His Asn Tyr Ser Gly Ile Lys Gly Asn His His Ala Leu Tyr Arg Asp
            20                  25                  30

Asp Phe Arg Lys Met Val Thr Thr Glu Cys Pro Gln Phe Val Gln Asn
        35                  40                  45

Lys Asn Thr Glu Ser Leu Phe Lys Glu Leu Asp Val Asn Ser Asp Asn
    50                  55                  60

Ala Ile Asn Phe Glu Glu Phe Leu Ala Leu Val Ile Arg Val Gly Val
65                  70                  75                  80

Ala Ala His Lys Asp Ser His Lys Glu
                85

<210>  4
<211>  270
<212>  DNA
<213>  Rattus norvegicus

<400>  4
atggcaactg aactggagaa ggccttgagc aacgtcattg aagtctacca caattattct      60

ggtataaaag ggaatcacca tgccctctac agggatgact tcaggaaaat ggtcactact     120

gagtgccctc agtttgtgca gaataaaaat accgaaagct tgttcaaaga attggacgtc     180

aatagtgaca acgcaattaa cttcgaagag ttccttgcgt tggtgataag ggtgggcgtg 240

gcagctcata aagacagcca caaggagtaa 270

<210> 5
<211> 93
<212> PRT
<213> Homo sapiens

<400> 5

Met Leu Thr Glu Leu Glu Lys Ala Leu Asn Ser Ile Ile Asp Val Tyr
1               5                   10                  15

His Lys Tyr Ser Leu Ile Lys Gly Asn Phe His Ala Val Tyr Arg Asp
            20                  25                  30

Asp Leu Lys Lys Leu Leu Glu Thr Glu Cys Pro Gln Tyr Ile Arg Lys
        35                  40                  45

Lys Gly Ala Asp Val Trp Phe Lys Glu Leu Asp Ile Asn Thr Asp Gly
    50                  55                  60

Ala Val Asn Phe Gln Glu Phe Leu Ile Leu Val Ile Lys Met Gly Val
65                  70                  75                  80

Ala Ala His Lys Lys Ser His Glu Glu Ser His Lys Glu
                85                  90

<210> 6
<211> 282
<212> DNA
<213> Homo sapiens

<400> 6
atgttgaccg agctggagaa agccttgaac tctatcatcg acgtctacca caagtactcc 60

ctgataaagg ggaatttcca tgccgtctac agggatgacc tgaagaaatt gctagagacc 120

gagtgtcctc agtatatcag gaaaaagggt gcagacgtct ggttcaaaga gttggatatc 180

aacactgatg gtgcagttaa cttccaggag ttcctcattc tggtgataaa gatgggcgtg 240

gcagcccaca aaaaaagcca tgaagaaagc cacaaagagt ag 282


<210> 7
<211> 113
<212> PRT
<213> Mus musculus

<400> 7

Met Ala Asn Lys Ala Pro Ser Gln Met Glu Arg Ser Ile Thr Thr Ile
1               5                   10                  15


Ile Asp Thr Phe His Gln Tyr Ser Arg Lys Glu Gly His Pro Asp Thr
            20                  25                  30


Leu Ser Lys Lys Glu Phe Arg Gln Met Val Glu Ala Gln Leu Ala Thr
        35                  40                  45

Phe Met Lys Lys Glu Lys Arg Asn Glu Ala Leu Ile Asn Asp Ile Met
    50                  55                  60

Glu Asp Leu Asp Thr Asn Gln Asp Asn Gln Leu Ser Phe Glu Glu Cys
65                  70                  75                  80

Met Met Leu Met Ala Lys Leu Ile Phe Ala Cys His Glu Lys Leu His
                85                  90                  95

Glu Asn Asn Pro Arg Gly His Gly His Ser His Gly Lys Gly Cys Gly
            100                 105                 110

Lys

<210>  8
<211>  342
<212>  DNA
<213>  Mus musculus

<400>  8
atggccaaca aagcaccttc tcagatggag cgcagcataa ccaccatcat cgacaccttc      60

catcaatact ctaggaagga aggacaccct gacaccctga gcaagaagga attcagacaa     120

atggtggaag cacagttggc aacctttatg aagaaagaga agagaaatga agccctcata     180

aatgacatca tggaggacct ggacacaaac caggacaatc agctgagctt tgaggagtgt     240

atgatgctga tggcaaagtt gatctttgcc tgtcatgaga agctgcatga gaacaaccca     300

cgtgggcatg gccacagtca tggcaaaggc tgtgggaagt aa                    342

<210> 9
<211> 113
<212> PRT
<213> Rattus norvegicus

<400> 9

Met Ala Ala Lys Thr Gly Ser Gln Leu Glu Arg Ser Ile Ser Thr Ile
1               5                   10                  15

Ile Asn Val Phe His Gln Tyr Ser Arg Lys Tyr Gly His Pro Asp Thr
            20                  25                  30

Leu Asn Lys Ala Glu Phe Lys Glu Met Val Asn Lys Asp Leu Pro Asn
        35                  40                  45

Phe Leu Lys Arg Glu Lys Arg Asn Glu Asn Leu Leu Arg Asp Ile Met
    50                  55                  60

Glu Asp Leu Asp Thr Asn Gln Asp Asn Gln Leu Ser Phe Glu Glu Cys
65                  70                  75                  80

Met Met Leu Met Gly Lys Leu Ile Phe Ala Cys His Glu Lys Leu His
                85                  90                  95

Glu Asn Asn Pro Arg Gly His Asp His Arg His Gly Lys Gly Cys Gly
            100                 105                 110

Lys

<210> 10
<211> 342
<212> DNA
<213> Rattus norvegicus

<400> 10
atggctgcca aaacaggatc tcagctggag cgcagcataa gcaccatcat caatgttttc 60

catcagtact ctaggaagta tggacatcct gacaccctga acaaggcgga attcaaagaa 120

atggtgaata aggacttgcc aaattttctg aagagggaga aaagaaatga aaatctccta 180

agagacatca tggaggacct ggacacaaac caggacaatc aactgtcctt tgaggagtgt 240

atgatgctga tgggaaagtt gatctttgcc tgtcatgaga agctgcatga gaacaaccca 300

cgtgggcatg accacaggca cggcaaaggc tgtgggaagt aa 342

<210> 11
<211> 116
<212> PRT
<213> Homo sapiens

<400> 11

Met Thr Cys Lys Met Ser Gln Leu Glu Arg Asn Ile Glu Thr Ile Ile
1               5                   10                  15

Asn Thr Pro His Glu His Gln Tyr Ser Val Lys Leu Gly His Pro Asp

            20                  25                  30

Thr Leu Asn Gln Gly Glu Phe Lys Glu Leu Val Arg Lys Asp Leu Gln
        35                  40                  45

Asn Phe Leu Lys Lys Glu Asn Lys Asn Glu Lys Val Ile Glu His Ile
    50                  55                  60

Met Glu Asp Leu Asp Thr Asn Ala Asp Lys Gln Leu Ser Phe Glu Glu
65                  70                  75                  80

Phe Ile Met Leu Met Ala Arg Leu Thr Trp Ala Ser His Glu Lys Met
                85                  90                  95

His Glu Gly Asp Glu Gly Pro Gly His His His Lys Pro Gly Leu Gly
            100                 105                 110

Glu Gly Thr Pro
        115

<210> 12
<211> 345
<212> DNA
<213> Homo sapiens

<400> 12
atgacttgca aaatgtcgca gctggaacgc aacatagaga ccatcatcaa caccttccac 60

caatactctg tgaagctggg gcacccagac accctgaacc agggggaatt caaagagctg 120

gtgcgaaaag atctgcaaaa ttttctcaag aaggagaata agaatgaaaa ggtcatagaa 180

cacatcatgg aggacctgga cacaaatgca gacaagcagc tgagcttcga ggagttcatc 240

atgctgatgg cgaggctaac ctgggcctcc cacgagaaga tgcacgaggg tgacgagggc 300

cctggccacc accataagcc aggcctcggg gagggcaccc cctaa 345

<210> 13
<211> 220
<212> PRT
<213> Artificial

<220>
<223> An artificially synthesized peptide sequence

<400> 13

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp
    210                 215                 220

<210>  14
<211>  660
<212>  DNA
<213>  Artificial

<220>
<223>  An artificially synthesized nucleotide sequence

<400>  14
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

<210>  15
<211>  63
<212>  DNA
<213>  Artificial

<220>
<223>  An artificially synthesized nucleotide sequence

<400>  15
atgcagacag acacactcct gctatgggta ctgctgctgt gggttccagg ttccactggt      60

gac                                                                   63